# FASCICULE DE BREVET EUROPEEN

(11) **EP 1 299 368 B1**
(45) Date de publication et mention de la délivrance du brevet: **31.08.2005**
(21) Numéro de dépôt: 01953176.3
(22) Date de dépôt: 26.06.2001
(51) Int. Cl.: C07D 301/12

(54) **PROCEDE DE FABRICATION D'OXIRANNE**
VERFAHREN ZUR HERSTELLUNG VON OXIRANEN
OXIRANE PRODUCTION METHOD

(30) Priorité: 28.06.2000 FR 0008356
(43) Date de publication de la demande: 09.04.2003
(73) Titulaire: SOLVAY (Société Anonyme), 1050 Bruxelles (BE)
(72) Inventeur: BALTHASART, Dominique, B-1120 Bruxelles (BE); STREBELLE, Michel, B-1150 Bruxelles (BE); CATINAT, Jean-Pierre, B-7131 Waudrez (BE)
(74) Mandataire: Vande Gucht, Anne
(86) Numéro de dépôt international: PCT/EP2001/007272
(87) Numéro de publication internationale: WO 2002/000636

(56) Documents cités:
- EP-A- 0 919 551
- EP-A- 0 930 308
- WO-A-98/28072
- WO-A-99/28029
- FR-A- 1 440 125

## Description

La présente invention concerne un procédé de fabrication d'oxyde de propylène par réaction de propylène avec un composé peroxydé dans un milieu liquide contenant un catalyseur solide. Elle concerne en particulier la fabrication d'oxyde de propylène par époxydation de propylène au moyen de peroxyde d'hydrogène.

Il est connu de fabriquer de l'oxyde de propylène par réaction entre le propylène et le peroxyde d'hydrogène en présence de silicalite de titane comme catalyseur. Par exemple, dans la demande de brevet EP 0 659 473 un tel procédé est réalisé dans un réacteur contenant un lit fixe du catalyseur.

Il est par ailleurs connu que l'activité des catalyseurs du type silicalite de titane dans ce type de fabrication chute au cours du temps. Dès lors, régulièrement, il est nécessaire de séparer le catalyseur du milieu réactionnel pour pouvoir le régénérer ou le remplacer.

Dans le procédé décrit dans la demande de brevet EP 0 659 473, le catalyseur présent sous la forme d'un lit fixe est difficile à sortir du réacteur pour le régénérer ou le remplacer.

Dans la demande internationale WO 98/28072, qui concerne la régénération de catalyseurs pour l'époxydation d'oléfines, des catalyseurs non-zéolitiques sont utilisés.

La demande de brevet EP 930308 concerne un procédé d'époxydation d'oléfines au moyen de peroxyde d'hydrogène en présence d'un catalyseur silicalite de titane que l'on a soumis à un traitement. L'oléfine peut être du propylène. L'époxydation peut être réalisée en présence d'un solvant. Un réacteur lit fluide peut être utilisé. Dans les exemples, l'époxydation est réalisée dans un réacteur agité dans lequel le catalyseur se trouve en suspension.

La demande e brevet EP 919551 concerne un produit à base d'épichlorhydrine obtenu par réaction de chlorure d'allyle avec un composé peroxydé en présence d'eau, d'un catalyseur et d'un diluant dans un réacteur à lit fixe, à lit transporté, à lit agité ou à lit fluidisé.

Le brevet FR 1440125 concerne l'époxydation d'oléfines en phase liquide, par l'eau oxygénée, et en présence d'une résine échangeuse de cations du type carboxylique à titre de catalyseur. L'époxydation peut être réalisée par simple passage des réactifs sur un lit de résine carboxylique ou par création d'un lit fluidisé constituant une zone de réaction au sein du réacteur.

La demande internationale WO 99/28029 concerne un catalyseur d'époxydation à base de zéolite au titane en forme de granules extrudés, qui peut être utilisé dans la synthèse d'oxirannes par réaction entre un composé oléfinique et un composé peroxydé.

La présente invention vise à remédier à cet inconvénient en fournissant un nouveau procédé de fabrication d'oxyde de propylène dans lequel le catalyseur est facile à séparer du milieu réactionnel. Un autre objectif de la présente invention est de fournir un procédé qui, lorsqu'il est réalisé à une échelle industrielle, permet d'évacuer de manière aisée la chaleur de réaction. Ceci permettrait d'opérer à une vitesse de réaction élevée ce qui conduit à une productivité élevée.

L'invention concerne dès lors un procédé continu de fabrication d'oxyde de propylène dans un réacteur contenant un milieu réactionnel liquide, selon lequel on fait réagir, dans le milieu réactionnel liquide, du propylène avec un composé peroxydé en présence de zéolite à titre de catalyseur solide et en présence d'un solvant; selon l'invention, le catalyseur solide est utilisé sous forme de particules ayant un poids spécifique apparent mesuré par écoulement libre dans l'air de 0,1 à 2 g/cm³ une partie au moins des particules se trouve, dans le réacteur, à l'état fluidisé, et le milieu réactionnel liquide contenant le propylène, le composé peroxydé, le solvant, l'oxyde de propylène formé et éventuellement des sous-produits, se déplace dans le réacteur du bas vers le haut de manière à créer un courant ascendant ayant une vitesse de montée telle que les particules de catalyseur soient fluidisées, la vitesse de montée du milieu réactionnel liquide étant de 0,01 à 10 m/min.

Une des caractéristiques essentielles du procédé selon l'invention réside dans la mise en oeuvre du catalyseur sous la forme de particules à l'état fluidisé. Le fait qu'un lit fluide de particules puisse être utilisé dans une réaction d'époxydation en milieu liquide de propylène avec un composé peroxydé en présence d'un solvant est surprenant. En effet, il n'est pas acquis que des particules de catalyseur d'époxydation supportent la fluidisation, car ces particules, par leur nature, sont fragiles et risquent d'être brisées ou cassées sous l'effet de la fluidisation. La demanderesse a maintenant constaté que ces particules, de manière surprenante, résistent à la fluidisation sans perte substantielle d'activité catalytique en présentant une faible attrition et un faible bris de grains. Le fait que ces particules puissent être mises en oeuvre à l'état fluidisé apporte comme avantage, par rapport au lit fixe, que le catalyseur est plus facile à sortir du réacteur pour le régénérer ou le remplacer. En outre, un régime en lit fluide assure un bon échange thermique et donc un meilleur contrôle de la température de réaction et assure une dispersion homogène du catalyseur dans le milieu réactionnel liquide.

Les principes de base du fonctionnement d'un régime en lit fluide sont décrits dans le "Perry's Chemical Engineers' Handbook, Sixth edition", 1984, pages 4-25, 4-26, 20-3 et de 20-58 à 20-75.

Dans le cadre de la présente invention, le terme "état fluidisé" signifie que les particules de catalyseur sont en mouvement continu, ce qui n'est pas le cas dans un lit fixe où le catalyseur reste immobile pendant toute la durée de la réaction. Toutefois, le mouvement des particules est limité car elles restent dans une zone du réacteur, que l'on appelle le lit fluide, qui est située entre une zone de distribution du fluide et une zone de dégagement des particules solides. Donc, en principe, les particules ne quittent pas la zone du lit fluide pendant la réaction, ce qui n'est pas le cas dans un lit transporté où les particules sont entraînées dans toutes les parties du réacteur.

La zone de distribution du fluide contient un distributeur qui a pour fonction d'éviter des courants préférentiels du fluide et donc d'assurer un courant homogène du fluide. Le distributeur consiste généralement d'une plaque de distribution ou d'une grille. La zone de dégagement des particules solides a pour fonction d'arrêter le mouvement des particules solides de catalyseur.

L'état fluidisé des particules de catalyseur est assuré par un fluide qui se déplace dans le réacteur du bas vers le haut de manière à créer un courant ascendant ayant une vitesse de montée telle que les particules de catalyseur soient fluidisées. De préférence, ce fluide est un liquide. Il est avantageusement constitué par le milieu réactionnel liquide qui contient le propylène, le composé peroxydé, le solvant, le plus souvent de l'eau, une partie de l'oxyde de propylène produit, et éventuellement des sous-produits formés pendant la réaction.

Plusieurs facteurs contribuent au bon fonctionnement du régime en lit fluide. On peut citer notamment le choix du distributeur, de la vitesse de montée du fluide, du poids spécifique des particules de catalyseur, du diamètre des particules de catalyseur, des dimensions du réacteur et de la hauteur du lit fluide. Tous ces paramètres dépendent l'un de l'autre. Par conséquent, pour réaliser un bon fonctionnement du lit fluide, il est nécessaire de sélectionner une combinaison optimale de paramètres qui puisse maintenir le catalyseur à l'état fluidisé pendant toute la durée de la réaction.

Dans le procédé selon l'invention, on peut mettre en oeuvre tout type de distributeur connu adéquat.

La vitesse de montée du fluide ascendant est supérieure ou égale à 0,01 m/min, en particulier supérieure ou égale à 0,05 m/min. Cette vitesse est inférieure ou égale à 10 m/min, en particulier inférieure ou égale à 5 m/min.

Les particules de catalyseur présentent un poids spécifique apparent mesuré par écoulement libre dans l'air supérieur ou égal à 0,1 g/cm³, en particulier supérieur ou égal à 0,5 g/cm³. Le poids spécifique apparent est inférieur ou égal à 2 g/cm³, plus particulièrement inférieur ou égal à 1 g/cm³.

Les particules de catalyseur ont couramment un diamètre supérieur ou égal à 100 µm, en particulier supérieur ou égal à 200 µm. Le diamètre moyen est en général inférieur ou égal à 5000 µm; en particulier inférieur ou égal à 2000 µm.

Le catalyseur contient avantageusement une fraction réduite de particules fines ayant un diamètre inférieur à 100 µm car ces fines sont facilement entraînées hors du lit fluide et provoquent ainsi une perte en catalyseur, un encrassement de l'installation ou l'apparition de réactions secondaires incontrôlées. En général, la fraction de fines est inférieure ou égale à 5 % en poids du catalyseur, en particulier inférieure ou égale à 2 % en poids, par exemple inférieure ou égale à 0,1 % en poids.

Les particules de catalyseur mises en oeuvre dans le procédé selon l'invention contiennent généralement un liant et un élément actif. La quantité de liant est généralement supérieure ou égale à 1 % en poids du catalyseur, en particulier supérieure ou égale à 10 %. La teneur en liant est le plus souvent inférieure ou égale à 90 % en poids du catalyseur, en particulier inférieure ou égale à 60 % en poids.

L'élément actif est une zéolite, et de manière préférée, une zéolite au titane. Par zéolite au titane, on entend désigner un solide contenant de la silice qui présente une structure cristalline microporeuse de type zéolite et dans laquelle plusieurs atomes de silicium sont remplacés par des atomes de titane. La zéolite au titane présente avantageusement une structure cristalline de type ZSM-5, ZSM-11, ZSM-12, MCM-41, ZSM-48. Elle peut aussi présenter une structure cristalline de type zéolite bèta, de préférence exempte d'aluminium. Les zéolites présentant une bande d'absorption infrarouge à environ 950-960 cm⁻¹ conviennent bien. Les zéolites au titane de type silicalite sont préférées. Celles répondant à la formule xTiO₂(1-x)SiO₂ dans laquelle x est de 0,0001 à 0,5 de préférence de 0,001 à 0,05, sont performantes. Des matériaux de ce type, connus sous le nom de TS-1, présentent une structure zéolitique cristalline microporeuse analogue à celle de la zéolite ZSM-5.

Le liant comprend généralement un ou plusieurs dérivés de silicium.

Les particules de catalyseur peuvent être obtenues par tour moyen connu, par exemple par extrusion, comme décrit dans la demande de brevet WO 99/28029 de la demanderesse, dont le contenu est incorporé par référence dans la présente demande de brevet, ou par un procédé en spray, comme décrit dans la demande de brevet WO 99/24164 de la demanderesse, dont le contenu est également incorporé par référence dans la présente demande de brevet.

Dans une première forme de réalisation du procédé selon l'invention, le réacteur est constitué de plusieurs réacteurs tubulaires disposés en parallèle dans un échangeur de chaleur, chaque réacteur contenant un lit fluide de particules de catalyseur. En général, les réacteurs tubulaires sont alimentés en parallèle par une source unique en milieu réactionnel liquide contenant le propylène, le composé peroxydé et le solvant. Cette source unique peut également contenir des tracés recyclées d'oxyde de propylène formé et/ou de sous-produits. L'échangeur de chaleur est avantageusement constitué d'une enceinte remplie de liquide de refroidissement, dans laquelle sont plongés les réacteurs tubulaires. Une solution alternative consiste à faire circuler dans ladite enceinte le liquide de refroidissement qui peut être maintenu à une pression suffisante pour ne pas changer d'état (et simplement s'échauffer) ou peut être partiellement vaporisé.

Cette première forme de réalisation s'avère particulièrement intéressante car elle permet d'assurer des conditions (notamment la perte de charge) équivalentes dans chaque réacteur tubulaire de manière plus aisée par rapport à un procédé qui utilise le catalyseur en lit fixe. En outre, elle permet d'opérer dans des tubes de réaction de taille réduite, même à l'échelle industrielle. Dans des réacteurs de taille réduite, il est plus facile d'obtenir une dispersion homogène du catalyseur car dans un grand réacteur la probabilité de.créer des courants préférentiels dans certaines parties du réacteur est plus élevée. Des réacteurs de taille réduite permettent également d'opérer à une vitesse de réaction plus élevée tout en évitant la formation de sous-produits. Il a en effet été constaté que l'oxiranne formé peut subir, dans le milieu réactionnel d'époxydation, des réactions secondaires d'hydrolyse et d'alcoolyse (méthanolyse lorsque le méthanol est utilisé comme solvant) pour former des sous-produits. Dans un réacteur de taille réduite, le contact entre l'oxyde de propylène formé et l'eau ou le solvant est minimalisé par rapport à un grand réacteur.

Dans une deuxième forme de réalisation du procédé selon l'invention le réacteur est constitué d'une enceinte unique contenant le milieu réactionnel liquide et le catalyseur à l'état fluidisé, dans laquelle sont plongés un ou plusieurs tubes disposés l'un à côté de l'autre et parcourus par un liquide de refroidissement. Une solution alternative consiste à faire circuler dans lesdits tubes le liquide de refroidissement qui peut être maintenu à une pression suffisante pour ne pas changer d'état (et simplement s'échauffer) ou peut être partiellement vaporisé.

Ces deux formes de réalisation permettent d'évacuer da manière aisée la chaleur de réaction formée au cours de l'époxydation par l'échauffement et/ou l'évaporation du liquide de refroidissement.

Le solvant mis en oeuvre dans le procédé selon l'invention peut être choisi parmi les alcools aliphatiques saturés, linéaires ou branchés. Le solvant alcoolique contient généralement jusqu'à 10 atomes de carbone, de préférence de 1 à 6 atomes de carbone. On peut citer à titre d'exemples le méthanol et l'éthanol. Le méthanol est préféré.

Le milieu réactionnel d'époxydation contient le plus souvent également de l'eau.

La quantité de solvant mise en oeuvre dans le procédé selon l'invention est généralement d'au moins 25 % en poids du milieu réactionnel liquide, en particulier d'au moins 40 % en poids, par exemple d'au moins 50 % en poids. Cette quantité ne dépasse habituellement pas 99 % en poids, en particulier pas 95 % en poids.

Le rapport molaire entre les quantités de propylène et de composé peroxydé engagées dans le procédé selon l'invention est généralement d'au moins 0.1, en particulier d'au moins 0,2, et de préférence d'au moins 0,5. Ce rapport molaire est le plus souvent d'au plus 100, en particulier d'au plus 50 et de préférence d'au plus 25.

Le procédé selon l'invention peut être réalisé en un seul réacteur ou en plusieurs réacteurs disposés en série. Dans un procédé en plusieurs réacteurs, il peut s'avérer avantageux d'introduire le composé peroxydé uniquement dans le premier réacteur, comme décrit dans la demande de brevet de la demanderesse déposée le même jour que la présente demande de brevet et intitulée "Procédé de fabrication d'oxiranne au moyen d'un composé peroxydé" (dont le contenu est incorporé par référence). En outre, chaque réacteur peut être suivi d'une colonne à distiller pour séparer l'oxyde de propylène formé du milieu réactionnel liquide, avant d'introduire celui-ci dans le réacteur suivant, comme décrit dans la demande de brevet de la demanderesse déposée le même jour que la présente demande de brevet et intitulée "Procédé de fabrication d'oxiranne comprenant la séparation de l'oxiranne du milieu réactionnel" (dont le contenu est incorporé par référence).

Dans le procédé selon l'invention, le composé peroxydé est généralement mis en oeuvre en une quantité d'au moins 0,005 mole par heure et par gramme de catalyseur, en particulier, d'au moins 0,01 mole par heure et par gramme de catalyseur. La quantité de composé peroxydé est habituellement inférieure ou égale à 25 moles par heure et par gramme de catalyseur et, en particulier, inférieure ou égale à 10 moles par heure et par gramme de catalyseur. Une préférence est montrée pour une quantité de composé peroxydé supérieure ou égale à 0,03 mole par heure et par gramme de catalyseur et inférieure ou égale à 2,5 mole par heure et par gramme de catalyseur.

Dans le procédé selon l'invention le composé peroxydé est avantageusement mis en oeuvre sous forme d'une solution aqueuse. En général, la solution aqueuse contient au moins 2 % en poids de composé peroxydé, en particulier au moins 5 % en poids. Elle contient le plus souvent au maximum 90 % en poids de composé peroxydé, en particulier 70 % en poids.

La température de la réaction entre le propylène et le composé peroxydé peut varier de 10 à 125 °C. Dans une variante avantageuse telle que décrite dans la demande de brevet EP99/08703 de la demanderesse, elle est supérieure à 35 °C pour remédier à la désactivation progressive du catalyseur. La température peut être supérieure ou égale à 40 °C et de préférence supérieure ou égale à 45 °C. Une température supérieure ou égale à 50 °C est tout particulièrement préférée. La température de réaction est de préférence inférieure à 100 °C.

Dans le procédé selon l'invention, la réaction entre le propylène et le composé peroxydé peut avoir lieu à pression atmosphérique. Elle peut également se dérouler sous pression. Généralement, cette pression n'excède pas 40 bar. Une pression de 20 bar convient bien en pratique.

Les composés peroxydés qui peuvent être utilisés dans le procédé selon l'invention sont les composés peroxydés contenant une ou plusieurs fonctions peroxyde (-OOH) qui peuvent libérer de l'oxygène actif et capables d'effectuer une époxydation. Les composés peroxydés inorganiques donnent de bons résultats. Le peroxyde d'hydrogène et les composés peroxydés qui peuvent produire du peroxyde d'hydrogène dans les conditions de la réaction d'époxydation conviennent bien. Le peroxyde d'hydrogène est préféré.

Lorsqu'on utilise du peroxyde d'hydrogène, il peut être intéressant de mettre en oeuvre dans le procédé selon l'invention une solution aqueuse de peroxyde d'hydrogène à l'état brut, c'est-à-dire non épurée. Par exemple, on peut mettre en oeuvre une solution obtenue par simple extraction avec de l'eau substantiellement pure du mélange issu de l'oxydation d'au moins une alkylanthrahydroquinone (procédé appelé "procédé AO auto-oxydation") sans traitement ultérieur de lavage et/ou de purification. Ces solutions brutes de peroxyde d'hydrogène contiennent généralement de 0,001 à 10 g/l d'impuretés organiques exprimées en COT (Carbone Organique Total). Elles contiennent habituellement des cations métalliques (tels que des métaux alcalins ou alcalino-terreux, comme le sodium) et des anions (tels que les phosphates, nitrates) en des teneurs de 0,01 à 10 g/l.

Dans une autre variante du procédé, on peut mettre en oeuvre une solution de peroxyde d'hydrogène produite par synthèse directe à partir d'oxygène et d'hydrogène en présence de méthanol.

Dans le procédé selon l'invention, un gaz n'ayant pas d'influence négative sur la réaction d'époxydation peut également être alimenté au réacteur. En effet, dans la demande de brevet WO 99/48883 (dont le contenu est incorporé par référence dans la présente demande de brevet), la demanderesse a trouvé qu'en introduisant un composé gazeux dans le milieu réactionnel à un débit suffisant pour permettre d'entraîner l'oxyde de propylène produit et de le sortir du réacteur en même temps que le composé gazeux, on diminue le temps de contact entre l'oxyde de propylène produit et le milieu réactionnel d'époxydation. On évite ainsi également la formation de sous-produits et on augmente la sélectivité de l'époxydation. Une autre variante consiste à séparer l'oxyde de propylène formé du milieu réactionnel liquide par distillation dans une colonne à distiller.

Dans le procédé selon l'invention il peut s'avérer intéressant de contrôler le pH de la phase liquide. Par exemple, il peut être intéressant de maintenir le pH de la phase liquide lors de la réaction entre le propylène et le composé peroxydé à une valeur de 4,8 à 6,5, par exemple par addition d'une base (hydroxyde de sodium) au milieu d'époxydation, comme recommandé dans la demande de brevet WO 99/48882 de la demanderesse (dont le contenu est incorporé par référence dans la présente demande de brevet).

La réaction entre le propylène et le composé peroxydé peut s'effectuer en présence d'un sel tel que le chlorure de sodium, comme décrit dans la demande de brevet WO EP99/08703 de la demanderesse (dont le contenu est incorporé par référence dans la présente demande de brevet).

Il peut être avantageux d'introduire le propylène dans le réacteur, dans lequel la réaction d'époxydation a lieu, à l'état dilué dans un ou plusieurs alcanes. Par exemple, on peut introduire dans le réacteur d'époxydation un fluide contenant le propylène et également au moins 10 % (en particulier 20%, par exemple au moins 30 %) en volume d'un ou plusieurs alcanes. Par exemple, le propylène peut être mélangé avec au moins 10 % en volume de propane lorsqu'on introduit dans le réacteur le propylène non converti recyclé. Il peut également s'agir d'une source de propylène incomplètement épurée en propane.

### Exemple 1

L'installation utilisée dans cet exemple d'époxydation du propylène (Pe) comprenait un réacteur en lit fluidisé avec boucle de recirculation de liquide. Ce réacteur était constitué d'un tube en verre de 1,5 cm de diamètre, à double enveloppe. Deux grilles servant à retenir le catalyseur étaient placées au haut et au bas du réacteur.

Le mélange réactionnel préalablement saturé en Pe, sous pression et constitué de méthanol (MeOH) + H₂O + H₂O₂ + Pe + oxyde de propylène (OP) a été envoyé, à un débit de 5 l/h, au réacteur où est confiné le catalyseur. L'OP formé a ensuite en partie été éliminé en phase gaz par stripping. La phase liquide résultante s'est partagée en un flux de sortie (débordement) et un flux de recirculation auquel se sont ajoutées les alimentations en H₂O₂ et MeOH et qui est retourné au réacteur, après avoir été à nouveau saturé en Pe.

Le catalyseur se présentait sous forme de billes de 0,4 - 0,6 mm constituées de silicalite de Ti dispersée à raison de 1/3 en poids dans une matrice de silice microporeuse. Elles avaient été préparées selon un procédé sol-gel en phase gazeuse.

Compte tenu du diamètre du réacteur, le débit du mélange réactionnel au travers du réacteur, qui a été fixé à 5 l/h, correspondait à une vitesse de passage tube vide de 0,47 m/min, soit une valeur supérieure à la vitesse minimale de fluidisation des billes, qui avoisinait 0,1 m/min. Cette vitesse minimale de fluidisation a été observée en raison de l'expansion du lit qu'elle occasionnait. La hauteur du lit catalytique est ainsi passé de 5 cm au repos à 7 cm en essai.

Après 347 h à 77 °C sous 8 bar, on a récupéré 4,441 g de catalyseur pour 4,500 g mis en oeuvre, soit une perte moyenne de 0,17 mg/h ou 0,004 %/h seulement.

## Revendications

1. Procédé continu de fabrication d'oxyde de propylène dans un réacteur contenant un milieu réactionnel liquide, selon lequel on fait réagir, dans le milieu réactionnel liquide, du propylène avec un composé peroxydé en présence de zéolite à titre de catalyseur solide et en présence d'un solvant, **caractérisé en ce que** le catalyseur solide est utilisé sous forme de particules ayant un poids spécifique apparent mesuré par écoulement libre dans l'air de 0,1 à 2 g/cm³ et **en ce qu'**une partie au moins des particules se trouve, dans le réacteur, à l'état fluidisé, et selon lequel, le milieu réactionnel liquide contenant le propylène, le composé peroxydé, le solvant, l'oxyde de propylène formé et éventuellement des sous-produits, se déplace dans le réacteur du bas vers le haut de manière à créer un courant ascendant ayant une vitesse de montée telle que les particules de catalyseur soient fluidisées, la vitesse de montée du milieu réactionnel étant de 0,01 à 10 m/min.

2. Procédé selon la revendication 1, **caractérisé en ce que** le catalyseur contient une fraction de particules fines ayant un diamètre inférieur à 100 µm qui soit inférieure ou égale à 5 % en poids du catalyseur.

3. Procédé selon la revendication 1 ou 2, **caractérisé en ce que** les particules de catalyseur ont un poids spécifique apparent de 0,5 à 1 g/cm³.

4. Procédé selon l'une quelconque des revendications 1 à 3, **caractérisé en ce que** les particules de catalyseur présentent un diamètre de 100 à 5000 µm.

5. Procédé selon l'une quelconque des revendications 1 à 4, **caractérisé en ce que** la vitesse de montée du milieu réactionnel liquide est de 0,05 à 5 m/min.

6. Procédé selon l'une quelconque des revendications 1 à 5, **caractérisé en ce que** le réacteur est constitué de plusieurs réacteurs tubulaires disposés en parallèle dans un échangeur de chaleur où ils sont alimentés par une source unique en milieu réactionnel liquide contenant l'oléfine, le composé peroxydé et le solvant.

7. Procédé selon la revendication 6, **caractérisé en ce que** la source unique contient également des traces recyclées d'oxiranne formé et/ou de sous-produits.

8. Procédé selon la revendication 6 ou 7, **caractérisé en ce que** la chaleur de la réaction est éliminée par circulation d'un fluide de refroidissement entourant les réacteurs tubulaires.

9. Procédé selon l'une quelconque des revendications 1 à 5, **caractérisé en ce que** le réacteur est constitué d'une enceinte unique contenant le milieu réactionnel liquide et le catalyseur à l'état fluidisé, dans laquelle sont plongés plusieurs tubes disposés l'un à côté de l'autre et parcourus par un fluide de refroidissement.

10. Procédé selon l'une quelconque des revendications 1 à 9, **caractérisé en ce que** le composé peroxydé est le peroxyde d'hydrogène, le catalyseur contient du silicalite de titane et le solvant est le méthanol.

11. Procédé selon l'une quelconque des revendications 1 à 10, **caractérisé en ce que** le milieu réactionnel contient également de l'eau.

## Patentansprüche

1. Kontinuierliches Verfahren zur Herstellung von Propylenoxid in einem ein flüssiges Reaktionsmilieu enthaltenden Reaktor, wonach in dem flüssigen Reaktionsmilieu Propylen mit einer Peroxidverbindung in Gegenwart von Zeolith als fester Katalysator und in Gegenwart eines Lösungsmittels zur Reaktion gebracht wird, **dadurch gekennzeichnet, daß** der feste Katalysator in Form von Teilchen mit einem scheinbaren spezifischen Gewicht, bestimmt durch freies Auslaufen in Luft, von 0,1 bis 2 g/cm³ verwendet wird und daß wenigstens ein Teil der Teilchen im Reaktor in einem fluidisierten Zustand vorliegt, und daß das das Propylen, die Peroxidverbindung, das Lösungsmittel, das gebildete Propylenoxid und gegebenenfalls Nebenprodukte enthaltende flüssige Reaktionsmilieu sich im Reaktor von unten nach oben bewegt, um einen aufsteigenden Strom mit einer solchen Steiggeschwindigkeit auszubilden, daß die Katalysatorteilchen fluidisiert werden, wobei die Steiggeschwindigkeit des Reaktionsmilieus 0,01 bis 10 m/min beträgt.

2. Verfahren nach Anspruch 1, **dadurch gekennzeichnet, daß** der Katalysator einen Anteil von Feinteilchen mit einem Durchmesser von kleiner als 100 µm enthält, der kleiner als oder gleich 5 Gew.-% des Katalysators ist.

3. Verfahren nach Anspruch 1 oder 2, **dadurch gekennzeichnet, daß** die Katalysatorteilchen ein scheinbares spezifisches Gewicht von 0,5 bis 1 g/cm³ aufweisen.

4. Verfahren nach einem der Ansprüche 1 bis 3, **dadurch gekennzeichnet, daß** die Katalysatorteilchen einen Durchmesser von 100 bis 5.000 µm aufweisen.

5. Verfahren nach einem der Ansprüche 1 bis 4, **dadurch gekennzeichnet, daß** die Steiggeschwindigkeit des flüssigen Reaktionsmilieus von 0,05 bis 5 m/min beträgt.

6. Verfahren nach einem der Ansprüche 1 bis 5, **dadurch gekennzeichnet, daß** der Reaktor aus mehreren, in einem Wärmetauscher parallel angeordneten rohrförmigen Reaktoren besteht, wo sie von einer einzigen Quelle mit dem das Olefin, die Peroxidverbindung und das Lösungsmittel enthaltenden flüssigen Reaktionsmilieu gespeist werden.

7. Verfahren nach Anspruch 6, **dadurch gekennzeichnet, daß** die einzige Quelle auch recyclierte Spuren an gebildetem Oxiran und/oder an Nebenprodukten enthält.

8. Verfahren nach Anspruch 6 oder 7, **dadurch gekennzeichnet, daß** die Reaktionswärme durch Zirkulierenlassen eines die Rohrreaktoren umgebenden Kühlfluids abgeführt wird.

9. Verfahren nach einem der Ansprüche 1 bis 5, **dadurch gekennzeichnet, daß** der Reaktor aus einem einzigen, das flüssige Reaktionsmilieu und den Katalysator im fluidisierten Zustand enthaltenden Behälter besteht, in den Seite an Seite mehrere Rohre eintauchen, die von einem Kühlfluid durchströmt werden.

10. Verfahren nach einem der Ansprüche 1 bis 9, **dadurch gekennzeichnet, daß** die Peroxidverbindung Wasserstoffperoxid ist, der Katalysator Titansilicalit enthält und das Lösungsmittel Methanol ist.

11. Verfahren nach einem der Ansprüche 1 bis 10, **dadurch gekennzeichnet, daß** das Reaktionsmilieu auch Wasser enthält.

## Claims

1. Continuous process for manufacturing propylene oxide in a reactor containing a liquid reaction medium, according to which propylene is reacted, in the liquid reaction medium, with a peroxide compound in the presence of a zeolite as solid catalyst and in the presence of a solvent, **characterized in that** the solid catalyst is used in the form of particles having an apparent specific weight, measured by free flow in air, of from 0.1 to 2 g/cm³, and **in that** at least a portion of the particles in the reactor are in fluidized form, and according to which the liquid reaction medium containing the propylene, the peroxide compound, the solvent, the propylene oxide formed and possibly by-products moves in the reactor from the bottom upwards so as to create an ascending stream having a rising speed such that the catalyst particles are fluidized, the rising speed of the liquid reaction medium being from 0.01 to 10 m/min.

2. Process according to claim 1, **characterized in that** the catalyst contains a fraction of fine particles having a diameter smaller than 100 µm, which is equal to or smaller than 5% wt of the catalyst.

3. Process according to Claim 1 or 2, **characterized in that** the catalyst particles have an apparent specific weight of from 0,5 to 1 g/cm³.

4. Process according to any one of Claims 1 to 3, **characterized in that** the catalyst particles have a diameter of from 104 to 5000 µm.

5. Process according to any one of Claims 1 to 4, **characterized in that** the rising speed of the liquid reaction medium is from 0,05 to 5 m/min.

6. Process according to any one of Claims 1 to 5, **characterized in that** the reactor consists of several tubular reactors arranged in parallel in a heat exchanger in which they are fed with a single source of liquid reaction medium containing the olefin, the peroxide compound and the solvent.

7. Process according to Claim 6, **characterized in that** the single source also contains recycled traces of the oxirane formed and/or of by-products.

8. Process according to Claim 6 or 7, **characterized in that** the heat of the reaction is removed by circulation of a coolant fluid surrounding the tubular reactors.

9. Process according to any one of Claims 1 to 5, **characterized in that** the reactor consists of a single chamber containing the liquid reaction medium and the catalyst in fluidized form, into which are immersed several tubes arranged side by side and through which passes a coolant fluid.

10. Process according to any one of Claims 1 to 9, **characterized in that** the peroxide compound is hydrogen peroxide, the catalyst contains titanium silicalite and the solvent is methanol.

11. Process according to any one of claims 1 to 10, **characterized in that** the liquid reaction medium also contains water.
